# EUROPÄISCHE PATENTANMELDUNG

(11) **EP 2 540 713 A1**
(43) Veröffentlichungstag der Anmeldung: **02.01.2013**
(21) Anmeldenummer: 11172144.5
(22) Anmeldetag: 30.06.2011
(51) Int. Cl.: C07D 313/00, C11B 9/00

(54) **Makrocyclische Lactone**

(71) Anmelder: BASF SE, 67056 Ludwigshafen (DE)
(72) Erfinder: Ebel, Klaus, 68623 Lampertheim (DE); Brunner, Bernhard, 69120 Heidelberg (DE); Stock, Christoph, 67158 Ellerstadt (DE); Pelzer, Ralf, 37669 Fürstenberg (DE)

(57) **Zusammenfassung**

Die vorliegende Erfindung betrifft neue makrocyclische Lactone, Verfahren zu deren Herstellung und deren Verwendung als Riechstoffe sowie Produkte enthaltend die neuen makrocyclischen Lactone.

## Beschreibung

Die vorliegende Erfindung betrifft neue makrocyclische Lactone, Verfahren zu deren Herstellung und deren Verwendung als Riechstoffe sowie Produkte enthaltend die neuen makrocyclischen Lactone.

Cyclische Enolether stellen bei der Synthese von makrocyclischen Lactonen, die als Riechstoffe verwendet werden, wichtige Zwischenprodukte dar. So wird in US 3,890,353 die Herstellung des gesättigten 15-Pentadecanolid (Exaltolide^{®}) der Formel (a) beschrieben, 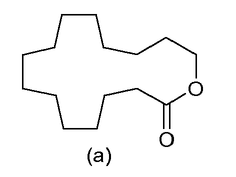 wobei als Ausgangsprodukt der cyclische Enolether 13-Oxa-1.12-didehydro-bicyclo[10.4.0] hexadecan der Formel (b) dient.

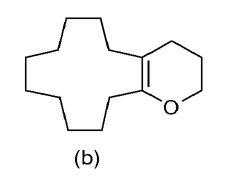

In US 5,266,559 wird beschrieben, dass bevorzugt die in der Synthese von 15-Pentadecanolid auftretenden Doppelbindungsisomere der Formel (c) mit einer Doppelbindung entweder in der 11- oder 12-Position jeweils in der trans-Konfiguration als moschusähnliche Riechstoffe verwendet werden können.

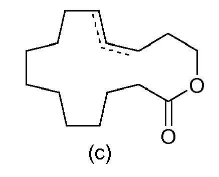

Neben den voran beschriebenen 16-Ring Lactonen sind auch 15-Ring Lactone als moschusähnliche Riechstoffe beschrieben. So werden in EP 0 862 911 gesättigte und ungesättigte 15-Ring Lactone der Formeln (d1) und (d2) beschrieben, wobei R gleich Methyl oder Wasserstoff ist.

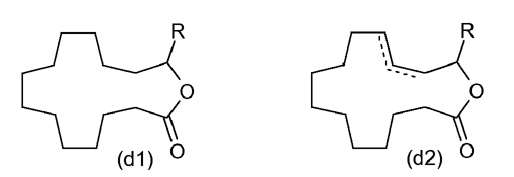

Die 15-Ring Lactone können ausgehend vom entsprechenden cyclischen Enolether der Formel (e) hergestellt werden.

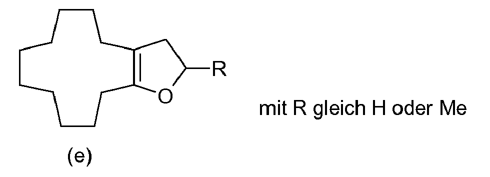

Die Herstellung von cyclischen Enolethern, die als Riechstoffe oder zur Herstellung von Riechstoffen geeignet sind, wird beispielsweise in GB 1266092, DE 2136496, US 3,890,353, DE 25 11 410, DE 29 06 296 oder JP 2010-95447 beschrieben.

Die Riechstoffe verarbeitende Industrie ist fortwährend auf der Suche nach neuen, interessanten Verbindungen, um ihre Kunden hinsichtlich deren Anforderungen und Wünschen geeignete Lösungen zur Verfügung stellen zu können. Insbesondere werden neue Verbindungen, die einen moschusartigen Geruch aufweisen, als Ersatzstoffe für den seltenen und teuren Moschus oder als Duftstoffkomponenten für Riechstoffkompositionen mit Moschuscharakter gesucht.

Ausgehend von diesem Stand der Technik bestand die Ausgabe darin, neue Riechstoffe zu finden.

Diese Aufgabe wird durch Verbindungen der Formeln (I) oder (II) oder Gemische derselben 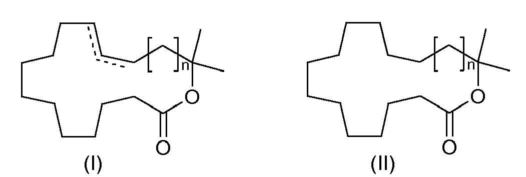 worin
n gleich null (0) oder eins (1) ist und
die gestrichelte Linie eine zusätzliche Doppelbindung in 11- oder 12-Position in cis- oder trans-Konfiguration bedeutet, gelöst.

Besonders bevorzugt sind Verbindungen der Formeln (I) wie vorangehend beschrieben, wobei es sich um mindestens eines der Doppelbindungsisomere der Formeln (Ia), (Ib), (Ic) oder (Id) oder um Gemische derselben 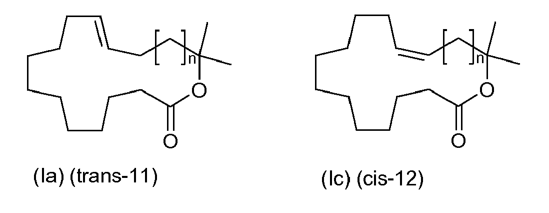 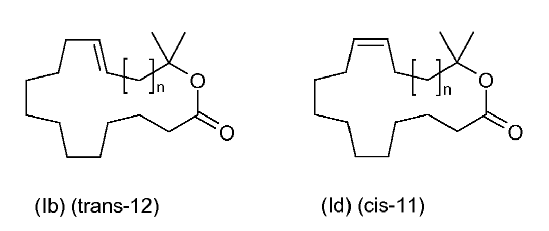 handelt und n wie in Formel (I) definiert ist

Besonders bevorzugt sind Zusammensetzung, die Gemische der oben beschriebenen Doppelbindungsisomere der Formeln (Ia), (Ib), (Ic) oder (Id) enthalten, wobei das Gemisch einen Gehalt von mindestens 60 Gew.-%, bevorzugt mindestens 70 Gew.-%, insbesondere mindestens 80 Gew.-% bis maximal 99,5 Gew.-% der Verbindung der Formel (Ia), also dem Lacton mit einer trans-konfigurierten Doppelbindung in Position 11, aufweist.

Die Verbindungen der Formeln (I) oder (II) oder Gemische derselben, insbesondere Isomerengemische enthalten wenigstens eines der Doppelbindungsisomere der Formeln (Ia), (Ib), (Ic) oder (Id) weisen im Vergleich zu den in der Einleitung beschriebenen Doppelbindungsisomeren der Formel (c) einen intensiveren und im Ganzen animalisch und abgerundeter wirkenden Geruch auf.

Ein weiterer Gegenstand der Erfindung ist auch ein Verfahren zur Herstellung der Verbindungen der Formeln (I) oder (II) oder der Verbindungen der Formeln (Ia), (Ib), (Ic) oder (Id) wie vorangehend beschrieben umfassend die Reaktionsschritte:
a) Alkylierung des Cyclododecanons der Formel (III) 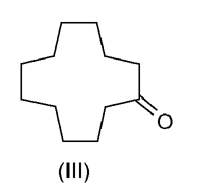 mit einer Verbindung der Formel (IVa) oder einer Verbindung der Formel (IVb) 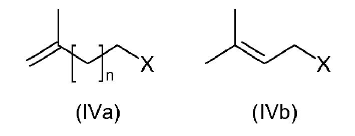 worin in Formel (IVa) und (IVb)
   - X: eine Abgangsgruppe ist und
   - n: gleich null (0) oder eins (1) ist,
   zu einer entsprechenden Verbindung der Formel (Va) oder der Formel (Vb), 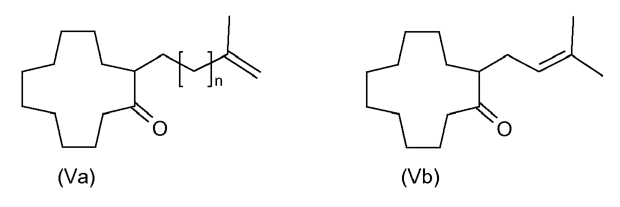 worin in Formel (Va) n gleich null (0) oder eins (1) ist;
b) Cyclisierung einer der Verbindungen der Formeln (Va) oder (Vb) zu einer entsprechenden Verbindung der Formel (VI); 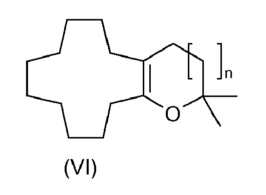 worin in Formel (VI) n gleich null (0) oder eins (1) ist;
c) Addition von H₂O₂ an die Doppelbindung der Verbindung der Formel (VI) unter Erhalt einer Verbindung der Formel (VII) und anschließende Übergangsmetall [ÜM] - katalysierte Fragmentierung der Verbindung der Formel (VII) zu Verbindungen der Formel (I) 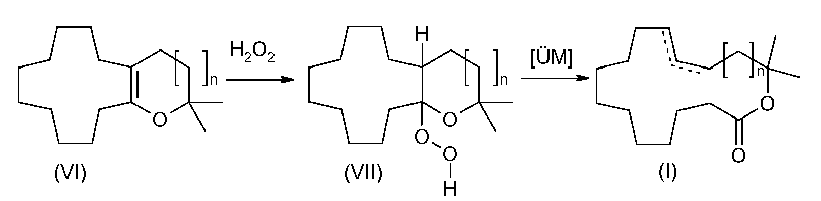 und gegebenenfalls
d) Hydrierung der Verbindungen der Formel (I) zu einer Verbindung der Formel (II) 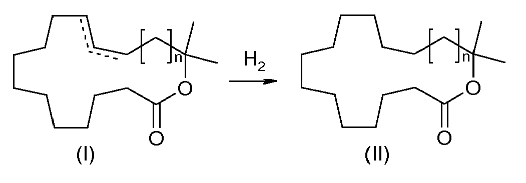 worin in den Formeln (I), (II) und (VII)
   - n: gleich null (0) oder eins (1) ist und
   die gestrichelte Linie in Formel (I) eine zusätzliche Doppelbindung in 11- oder 12-Position in cis- oder trans-Konfiguration bedeutet.

Als Schlüsselschritt des Verfahrens erwies sich Verfahrensschritt b), worin in einer bevorzugten Ausführungsform die Cyclisierung in Gegenwart einer Brönstedt- oder Lewis-Säure als Reaktivdestillation durchgeführt wurde.

Daher ist auch ein weiterer Gegenstand der Erfindung ein Verfahren zur Herstellung der Verbindungen der Formeln (I) oder (II) oder der Verbindungen der Formeln (Ia), (Ib), (Ic) oder (Id) wie vorangehend beschrieben umfassend als einen der Reaktionsschritte eine Cyclisierung einer Verbindung der Formel (Va) oder der Formel (Vb), 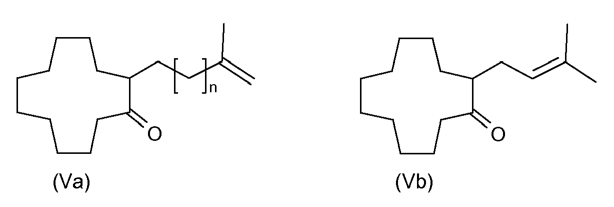 zu einer entsprechenden Verbindung der Formel (VI); 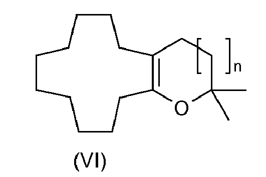 worin in Formeln (Va) und (VI) n gleich null (0) oder eins (1) ist, dadurch gekennzeichnet, dass die Cyclisierung in Gegenwart einer Brönstedt- oder Lewis-Säure als Reaktivdestillation durchgeführt wird, wobei die gebildete Verbindung der Formel (VI) destillativ aus dem Reaktionsgemisch von der Verbindung der Formel (Va) oder der Formel (Bb) abgetrennt wird.

In Verfahrensschritt a) des erfindungsgemäßen Verfahrens wird Cyclododecanon (CDon) der Formel (lll), ein großtechnisch produziertes Zwischenprodukt, 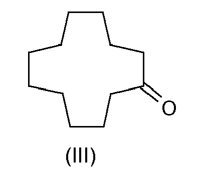 mit einer Verbindung der Formel (lVa) oder einer Verbindung der Formel (lVb) alkyliert 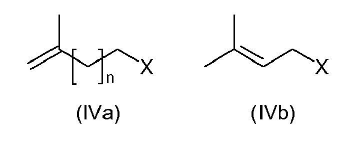 worin in Formel (lVa) und (lVb)
- X: eine Abgangsgruppe ist und
- n: gleich null (0) oder eins (1), bevorzugt null (0) ist
zu einer entsprechenden Verbindung der Formel (Va) oder der Formel (Vb), 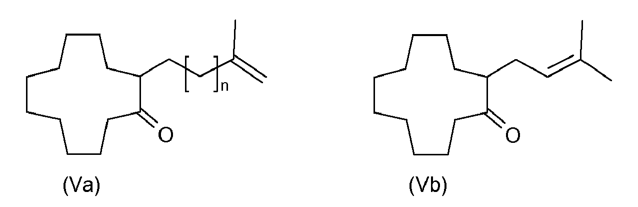 worin in Formel (Va) n gleich null (0) oder eins (1), bevorzugt null (0) ist, umgesetzt.

Bei der Abgangsgruppe X handelt es sich um dem Fachmann bekannte funktionelle Gruppen, wie sie üblicherweise in nukleophilen Substitutionsreaktionen eingesetzt werden. Nicht einschränkende Beispiele für X sind Cl, Br, I, OSO₂C₆H₄Me, OSO₂Me, OSO₂CF₃, OSO₂C₄F₉Me, OSO₂C₆H₄Me, OSO₂C₆H₄Me. Bevorzugt ist X gleich Cl oder Br, insbesondere Cl.

Die Monoalkylierung des Cyclododecanons (CDon) kann nach dem Fachmann bekannten Verfahren durchgeführt werden. Beispielsweise kann CDon zunächst mit Chlorameisensäurealkylester zum entsprechenden beta-Ketocarbonsäureester umgesetzt werden, der nach Alkylierung mit einer Verbindung der Formel (IVa) oder einer Verbindung der Formel (lVb), nachfolgender Verseifung und Decarboxylierung je nach eingesetztem Alkylierungsreagenz zu einer Verbindung der Formel (Va) oder (Vb) umgesetzt wird, so wie es beispielsweise in GB 1266092 prinzipiell beschrieben wird.

Bevorzugt wird die Monoalkylierung des Cyclododecanons (CDon) durch direkte Umsetzung von CDon mit einem Alkylierungsmittel der Formel (IVa) oder der Formel (IVb) in Gegenwart einer Base und eines Phasentransferkatalysators (PTC) durchgeführt, so wie beispielsweise in DE 2916418 beschrieben. Als PTC werden vorzugsweise preiswerte Tetraalkylammoniumhalogenide, bei beispielsweise Tetrabutylammoniumiodid, und als Base preiswerte Alkali- oder Erdalkalimetallhydroxide, insbesondere Natriumhydroxid eingesetzt.

In Verfahrensschritt b) des erfindungsgemäßen Verfahrens wird eine Verbindung der Formel (Va) oder der Formel (Vb) zu einer entsprechenden Verbindung der Formel (VI) 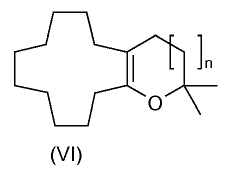 cyclisiert, worin in Formel (VI) n gleich null (0) oder eins (1) ist. Die Verbindung der Formel (Vb) cyclisiert zu einer Verbindung der Formel (VI) mit n gleich 1.

Bevorzugt findet die Cyclisierung in Gegenwart einer Brönstedt- oder Lewis-Säure, vorzugsweise einer Brönstedt-Säure statt, wobei die Reaktion als Reaktivdestillation durchgeführt wird, wobei die gebildeten cyclischen Enolether der Formel (VI) destillativ aus dem Reaktionsgemisch von Verbindungen der Formel (Va) oder der Formel (Vb) abgetrennt werden.

Die im Folgenden beschriebenen bevorzugten Ausführungsformen bezüglich Verfahrensschritt b) beziehen sich auch auf das oben beschriebene Verfahren umfassend die als Reaktivdestillation geführte Cyclisierung in Gegenwart einer Brönstedt- oder Lewis-Säure.

Besonders bevorzugt ist in Verbindungen der Formel (Va) n gleich null (0), was zur Bildung des 5-gliedrigen Cycloenolethers der Formel (VI) mit n gleich 0 (null) führt.

Bei den bevorzugt in Verfahrensschritt b) verwendbaren Brönstedt-Säuren handelt es sich sowohl um organische als auch um anorganische Säuren. Bevorzugt werden Brönstedt-Säuren, die selbst nicht mit der Verbindung der Formel (Va) oder der Formel (Vb) reagieren können, das heißt in einer Reaktion verbraucht werden, sondern lediglich als Protonenquelle für eine durch Protonen katalysierte chemische Reaktion dienen. Nicht einschränkende Beispiele für besonders geeignete Brönstedt-Säuren sind Schwefelsäure, Phosphorsäure, Methansulfonsäure, p-Toluolsulfonsäure, stark saure Ionenaustauscher, Tetrafluoroborsäure, Trifluoressigsäure, Ameisensäure oder Oxalsäure.

Als Lewis-Säure können bevorzugt in Verfahrensschritt b) beispielsweise Aluminiumtrichlorid, Zinntetrachlorid, Titantetrachlorid, Zirkoniumtetrachlorid, Eisentrichlorid oder Nickeldichlorid eingesetzt werden.

Die Menge der Brönstedt- oder Lewis-Säure, die bevorzugt in Verfahrensschritt b) verwendet wird, kann in einem breiten Bereich variiert werden. Prinzipiell kann das molare Verhältnis der Brönstedt- oder Lewis-Säure zur Verbindung der Formel (Va) oder der Formel (Vb) größer, gleich oder kleiner als 1 sein. Prinzipiell reichen Spuren von Säure, um die Cyclisierung zu katalysieren.

Bevorzugt ist in Verfahrensschritt b) das molare Verhältnis der Brönstedt- oder Lewis-Säure, insbesondere der Brönstedt-Säure zur Verbindung der Formel (Va) oder der Formel (Vb) nicht größer als 1, besonders bevorzugt nicht größer als 0,15, ganz besonders bevorzugt zwischen 0,1 und 0,0005, insbesondere zwischen 0,07 und 0,001.

Bevorzugt wird in Verfahrensschritt b) die Cyclisierung in Gegenwart einer Brönstedtsäure durchgeführt. Bevorzugt werden dabei Brönstedtsäuren mit einem pKₛ-Wert von kleiner 5, besonders bevorzugt kleiner als 2,5, insbesondere kleiner als 0 eingesetzt. Ganz besonders bevorzugt liegt der pKₛ-Wert der Brönstedtsäure zwischen -1,5 und -11.

Eine Reaktivdestillation ist ein dem Fachmann prinzipiell bekanntes chemisches Verfahren, bei dem eine ein- oder mehrstufige Destillation mit einer chemischen Reaktion, im vorliegenden Fall einer Cyclisierung, verbunden wird. Das Reaktionsprodukt, im vorliegenden Fall ein cyclischer Enolether der Formel (Vl), wird laufend durch Destillation vom Ausgangsprodukt, einem Keton, abgetrennt.

Bevorzugt wird die Reaktivdestillation bzw. die Cyclisierungsreaktion in einem Temperaturbereich zwischen 50 °C und 300 °C, besonders bevorzugt zwischen 80 °C und 200 °C durchgeführt.

In Abhängigkeit von den Siedepunkten der zu trennenden Verbindungen kann der Fachmann üblicherweise unmittelbar oder nach wenigen Versuchen bezüglich der verwendbaren Destillationskolonnen, dem notwendigen Trennleistungsvermögen einer solchen Kolonne sowie den Destillationsparametern wie beispielsweise Druck, Temperatur und Rücklaufverhältnis geeignete Maßnahmen ergreifen, beziehungsweise eine geeignete Auswahl treffen, um Verfahrensschritt b) in der gewünschten Weise durchführen zu können.

In Verfahrensschritt c) des erfindungsgemäßen Verfahrens wird die Addition von H₂O₂ an die Doppelbindung der Verbindung der Formel (VI) unter Erhalt einer Verbindung der Formel (VII) und anschließende Übergangsmetall [ÜM] - katalysierte Fragmentierung der Verbindung der Formel (VII) zu Verbindungen der Formel (I) durchgeführt, 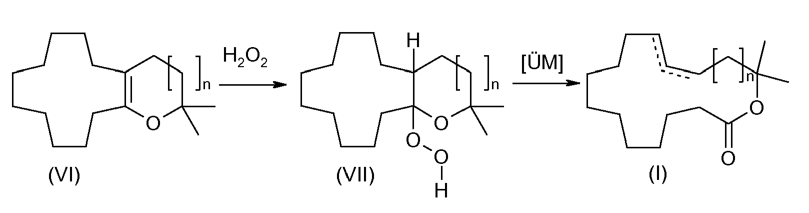 worin n gleich null (0) oder eins (1) ist und die gestrichelte Linie in Formel (I) eine zusätzliche Doppelbindung in 11- oder 12-Position in cis- oder trans-Konfiguration bedeutet.

Die Bedingungen für die Addition von H₂O₂ an die Doppelbindung der Verbindung der Formel (VI) und die anschließende Übergangsmetall-katalysierte Fragmentierung der Verbindung der Formel (VII) zu Verbindungen der Formel (I) sind prinzipiell aus der Literatur bekannt und werden beispielsweise in EP 0 862 911 und der dort zitierten Literatur wie beispielsweise einem Artikel von S.L. Schreiber, J. Am. Chem. Soc. 102 (1980), 6163 beschrieben.

In Verfahrensschritt c) wird die Fragmentierung der Verbindung der Formel (Vll) zur Verbindung der Formel (I) besonders bevorzugt in Gegenwart von Cu(II)- oder Fe(II)-Salzen durchgeführt.

Bei den in Verfahrensschritt c) gebildeten Verbindungen der Formeln (I) handelt es sich insbesondere um die Doppelbindungsisomere der Formeln (Ia), (Ib), (Ic) oder (Id) oder um Gemische derselben.

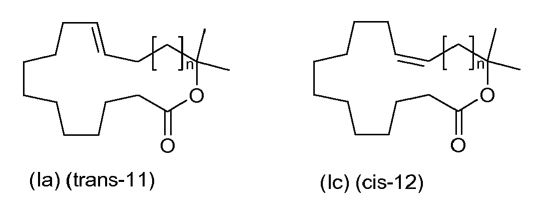

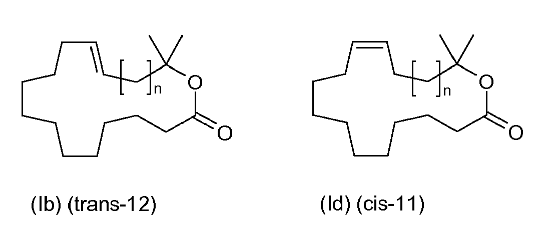

Bevorzugt werden in Verfahrensschritt c) überwiegend die trans-konfigurierten Doppelbindungsisomere der Formeln (Ia) und (Ib) gebildet. Daneben beobachtet man die Bildung geringer Mengen der gesättigten Verbindungen der Formel (II). Das molare Verhältnis der trans-konfigurierten Doppelbindungsisomere der Formeln (Ia) und (Ib) zu den cis-konfigurierten Doppelbindungsisomeren der Formeln (Ic) und (Id) ist vorzugsweise größer als 2, besonders bevorzugt größer als 3, insbesondere größer als 4. In Verfahrensschritt c) wird insbesondere das trans-konfigurierte Doppelbindungsisomer der Formel (Ia) gebildet, also das Lacton, bei dem die Doppelbindung in Position 11 liegt.

Der optionale Verfahrensschritt d) stellt die Hydrierung der Verbindungen der Formel (I) zu einer Verbindung der Formel (II) dar, 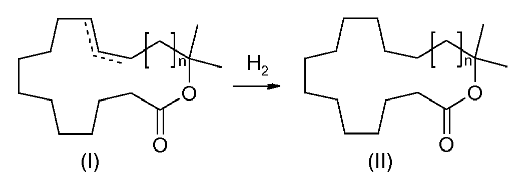 worin n gleich null (0) oder eins (1) ist und die gestrichelte Linie in Formel (l) eine zusätzliche Doppelbindung in 11- oder 12-Position in cis- oder trans-Konfiguration bedeutet.

Bevorzugt wird in Verfahrensschritt d) die Hydrierung mit Wasserstoff in Gegenwart eines Übergangsmetallkatalysators durchgeführt.

Die verwendbaren Übergangsmetallkatalysatoren und Reaktionsbedingungen für die Hydrierung der Doppelbindung in den Verbindungen der Formel (I) sind dem Fachmann prinzipiell bekannt. In US 3,890,353 wird beispielsweise die Hydrierung von 15-Pentadec-(11 und 12)-enolid in Gegenwart von Raney-Nickel beschrieben.

Die erfindungsgemäßen Verbindungen der Formeln (I) und/oder (II) oder der Verbindungen der Formeln (Ia), (Ib), (Ic) und/oder (Id) weisen wie vorangehend beschrieben einen moschusartigen Geruch auf.

Ein weiterer Gegenstand der vorliegenden Erfindung ist auch die Verwendung der Verbindungen der Formeln (I) und/oder (II) oder der Verbindungen der Formeln (Ia), (Ib), (Ic) und/oder (Id) wie vorangehend beschrieben als Riechstoffe.

Die erfindungsgemäßen Verbindungen der Formeln (I) und/oder (II) oder der Verbindungen der Formeln (Ia), (Ib), (Ic) und/oder (Id) wie voran stehend beschrieben können nicht nur allein als Riechstoff eingesetzt werden, sondern werden bevorzugt in Kombination mit bekannten Duftstoffen eingesetzt, wie sie beispielsweise in K. Bauer, D. Garbe und H. Surburg, Common Fragrance and Flavor Materials, 4th Edition, Wiley-VCH, Weinheim 2001 beschrieben werden.

Ein weiterer Gegenstand der vorliegenden Erfindung ist somit auch die Verwendung der Verbindungen der Formeln (I) und/oder (II) oder der Verbindungen der Formeln (Ia), (Ib), (Ic) und/oder (Id) wie voran stehend beschrieben als Riechstoff zusammen mit mindestens einem weiteren Riechstoff in einer Riechstoffkomposition.

Ebenfalls Gegenstand der vorliegenden Erfindung sind Riechstoffkomposition enthaltend mindestens eine der Verbindungen der Formeln (I) und/oder (II) oder der Verbindungen der Formeln (Ia), (Ib), (Ic) und/oder (Id) wie voran stehend beschrieben zusammen mit mindestens einem weiteren Riechstoff.

Als weiterer Riechstoff werden bevorzugt Riechstoffe eingesetzt, die einen moschusähnlichen Geruch, eine Zitrusnote, eine holzige Note, ein orientalische Note oder eine Ledernote aufweisen.

Die erfindungsgemäßen Verbindungen der Formeln (I) und/oder (II) oder der Verbindungen der Formeln (Ia), (Ib), (Ic) und/oder (Id) wie voran stehend beschrieben können einerseits in der Feinparfümerie eingesetzt werden, andererseits können sie aber auch zur Parfümierung unterschiedlicher Produkte oder Artikel verwendet werden, wie beispielsweise zur Parfümierung von Kosmetika wie Cremes, Lotionen, Aerosolen, Toilettenseifen, technischen Artikeln, Waschmitteln, Weichspülern, Desinfektionsmitteln oder Textilbehandlungsmitteln verwendet werden.

Gegenstand der vorliegenden Erfindung sind daher auch ein parfümiertes oder aromatisiertes Produkt oder Artikel enthaltend organoleptisch aktive Mengen mindestens einer der Verbindungen der Formeln (I) und/oder (II) oder der Verbindungen der Formeln (Ia), (Ib), (Ic) und/oder (Id) wie voran stehend beschrieben.

Die Erfindung wird durch folgende, die Erfindung jedoch nicht einschränkende Beispiele erläutert.

### Beispiel 1:

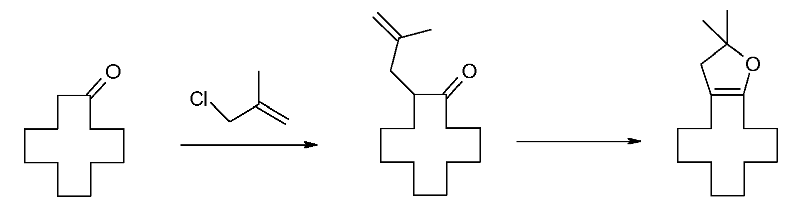

### Allyllierung:

CDon (364,6 g), Toluol (360 ml), Tetrabutylammoniumiodid (7,6 g) und Natronlauge (50%ig, 480 g) wurden in den Reaktor eingefüllt und unter Rühren (400 UpM) auf 90 °C aufgeheizt. Bei 90 °C Innentemperatur wurde mit der Dosierung von Methallylchlorid
(362,2 g) begonnen, wobei die Temperatur im Reaktor abfiel und Rückfluss einsetzte. Gesamtdosierdauer: 3 h. Das zweiphasige Reaktionsgemisch wurde dann über Nacht bei 94 °C gerührt. Danach wurde die Reaktionslösung auf RT abgekühlt. Bei 65 °C wurden dabei 500 ml Wasser zugegeben, um den angefallenen Feststoff in der wässrigen Phase zu lösen. Nach der Phasentrennung wurde die organische Phase zweimal mit 500 ml Wasser gewaschen. Danach wurde die organische Phase noch mit 500 g 10%iger Schwefelsäure gewaschen. Die wässrigen Phasen wurden jeweils verworfen.

### Cyclisierung:

### Brönstedt-Säure-Katalyse

In einem 1000 ml Dreihalskolben mit 30 cm Sulzerkolonne und Normag-Kolonnenkopf wurden 1006 g Methallylcyclododecanon vorgelegt und mit 20 g konzentrierter Schwe-felsäure versetzt. Es wurde ein Vakuum von 1 mbar angelegt, die Ölbadtemperatur wurde auf 140 °C erhöht. Bei einer Sumpftemperatur von 128-135 °C wurde der Bicyclus langsam aus dem Reaktionsgemisch herausdestilliert (Kopftemperatur 91-96 °C). Insgesamt konnten 866,35 g Produkt aus dem Reaktionsgemisch herausdestilliert werden.

### Lewis-Säure-Katalyse

In einem 500 ml Destillationskolben mit 30 cm Füllkörperkolonne (3 mm Drahtringe) und Normag-Kolonnenkopf wurden 200 g 2-(2-Methallyl-)cyclododecanon vorgelegt und mit 2 g Aluminiumchlorid versetzt. Es wurde ein Vakuum von 2 mbar angelegt, die Ölbadtemperatur wurde langsam auf 175 °C erhöht. Bei einer Sumpftemperatur von 153-156 °C wurde das Produkt aus dem Reaktionsgemisch herausdestilliert (Kopftemperatur 121-123 °C). Insgesamt konnten 140,5 g Produkt aus dem Reaktionsgemisch herausdestilliert werden.

### Beispiel 2:

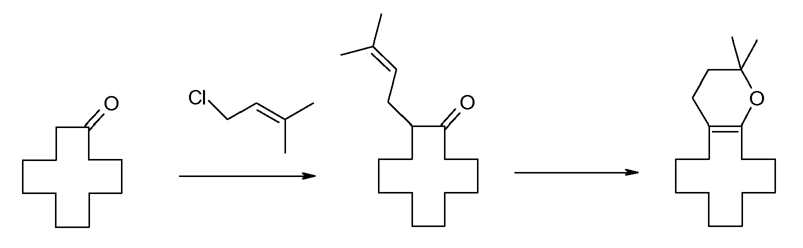

CDon (364,6 g), Toluol (360 ml), Tetrabutylammoniumiodid (7,6 g) und Natronlauge (50%ig, 480 g) wurden in den Reaktor eingefüllt und unter Rühren (400 UpM) auf 90 °C aufgeheizt. Bei 90 °C Innentemperatur wurde mit der Dosierung von 1-Chlor-3-methyl-2-buten (313,7 g) begonnen. Die Temperatur wurde während der gesamten Zugabe bei 90 °C gehalten. Gesamtdosierdauer: 3 h. Das zweiphasige Reaktionsgemisch wurde für 5 h bei 90 °C nachgerührt. Danach wurde die Reaktionslösung auf RT abgekühlt. Bei 65 °C wurden 500 ml Wasser zugegeben, um den angefallenen Feststoff in der wässrigen Phase zu lösen. Nach der Phasentrennung wurde die organische Phase zweimal mit 500 ml Wasser gewaschen. Danach wurde die organische Phase noch mit 500 g 10%iger Schwefelsäure gewaschen. Die wässrigen Phasen wurden jeweils verworfen.

Zu 278 g des Zwischenproduktes wurden 7 g konz. Schwefelsäure gegeben und dann die Lösung in einen 1l Destillationskolben umgefüllt und in einer 70 cm Füllkörperkollonne (3 mm Metalraschigringe) mit Rücklaufteiler bei einer Sumpftemperatur von 185°C einer Kopftemperatur von 130-135 °C einem Druck von 3 mbar und einem Rücklaufverhältnis von 40:1 bis 60:1 destilliert. 191,7 g des Produktes konnten aus der Reaktionsmischung herausdestilliert werden.

### Beispiel 3: Herstellung von 14-Dimethyltetradecenolid

### a) Hydroperoxid-Bildung

764 g 14-Dimethyl-13-oxabicyclo[10.3.0]-pentadec-[1 (12)]-en (3,2 mol) aus Beispiel 1 wurden zusammen mit 1900 g Propionsäure und 8,0 g konzentrierter H₂SO₄ in einem 1 Liter Dreihalskolben vorgelegt und auf 0 °C abgekühlt. Anschließend wurden 295 g einer 50 %-igen H₂O₂-Lösung innerhalb von 45 Minuten langsam zugetropft. Das Reaktionsgemisch wurde für 2 Stunden bei 0-5 °C nachgerührt, wobei sich eine weiße Suspension bildete. Die Schwefelsäure wurde im Anschluss durch die Zugabe von
63 g einer 10 %-igen NaOH-Lösung neutralisiert.

### b) Fragmentierung

In einer 1 Liter Destillationsapparatur wurden 300 g Polyethylenglycol 400 (käuflich erhältlich als Lutrol® E400) zusammen mit 4,7 g Cu(OAc)₂ vorgelegt. Das Gemisch wurde bei einem Druck von 20 mbar auf 100-110 °C erwärmt. Mittels einer Taumelkopfpumpe wurde die gekühlte Hydroperoxid-Lösung aus a) innerhalb von 2-3 Stunden in die PEG-Lösung dosiert. Parallel hierzu wurde ein Azeotrop aus Propionsäure und Wasser kontinuierlich aus der Reaktionslösung abdestilliert. Der auf diese Weise erhaltene Reaktionsaustrag wurde zunächst bei 5 mbar und einer Sumpftemperatur von 140 °C über eine einfache Brücke destilliert. Anschließend wurde das Destillat aus der Grobdestillation über eine 20 cm Füllkörperkolonne bei einem Druck von 0,5-1 mbar und einer Übergangstemperatur von 85-99 °C feindestilliert. Auf diese Weise wurden 540 g eines 14-Dimethyltetradecenolid-Gemisches mit einer Reinheit von >95 % erhalten. Die Isomerenverteilung des 14-Dimethyltetradecenolid-Gemisches wurde mittels NMR-Spektroskopie, GC-MS und GC-lR zu
82 % trans-14-Dimethyltetradec-11-en-14-olid (Retentionszeit: 28,31 Minuten)
2 % trans-14-Dimethyltetradec-12-en-14-olid (Retentionszeit: 27,35 Minuten)
11 % cis-14-Dimethyltetradec-11-en-14-olid (Retentionszeit: 28,49 Minuten)
2 % cis-14-Dimethyltetradec-12-en-14-olid (Retentionszeit: 27,30 Minuten)
3 % Dimethyltetradecan-14-olid (Retentionszeit: 27,61 Minuten)
bestimmt.
GC-MS: 30 m Säule DB-WAX ID: 0,32 mm FD: 0,25 µm; Temperaturprogramm: 50 °C mit 5 °C pro Minute auf 240 °C und 25 Minuten isotherm.

NMR (¹³C, 150 MHz, C₆D₆) (ppm): 172,4 (s, cis + trans), 133,6 (d, trans), 132,5 (d, cis), 126,2 (d, trans), 124,8 (d, cis), 81,1 (s, trans), 80,8 (s, cis), 43,6 (t, trans), 38,5 (t, cis), 35,0 (t, cis), 34,8 (t, trans), 30,9 (t, trans), 28,1 (t, cis), 27,7 (t, trans), 27,5 (t, trans), 27,3 (t, trans), 26,8 (t, cis), 26,6 (q, cis), 26,57 (t, cis), 26,4 (q, trans), 26,3 (t, trans), 26,2 (t, trans), 26,15 (t, cis), 26,1 (t, cis), 25,7 (t, cis), 25,6 (t, cis), 25,5 (t, cis), 25,2 (t, trans), 24,9 (t, trans), 23,7.
NMR (1H, 600 MHz, C₆D₆) δ (ppm) trans-14-Dimethyltetradecen-11-olid: 4,40-5,52 (1H, dt, J₁ = 15,6 Hz, J₂ = 6,95 Hz); 5,3-5,4 (1H, dt, J₁ = 15,6 Hz, J₂ = 6,7 Hz); 2,47 (2 H, d); 2,15 (2 H, t), 2,05 (2 H, m), 1,2-1,6 (m, 12 H), 1,41 (6 H, s).

### Beispiel 4: Vorschrift zur Herstellung von 15-Dimethylpentadecenolid

### a) Hydroperoxid-Bildung

50 g 14-Dimethyl-13-oxabicyclo[10.4.0]-hexadec-[1 (12)]-en aus Beispiel 2 werden zusammen mit 115 g Propionsäure und 0,6 g konzentrierter H₂SO₄ in einem 1 Liter Dreihalskolben vorgelegt und auf 0 °C abgekühlt. Anschließend werden 17,8 g einer 50 %-igen H₂O₂-Lösung innerhalb von 45 Minuten langsam zugetropft. Das Reaktionsgemisch wird für 2 Stunden bei 0-5 °C nachgerührt, wobei sich eine weiße Suspension bilden sollte. Die Schwefelsäure wird im Anschluss durch die Zugabe von 5 g einer 10 %-igen NaOH-Lösung neutralisiert.

### b) Fragmentierung

In einer 1 Liter Destillationsapparatur werden 100 g Polyethylenglycol 400 (käuflich erhältlich als Lutrol® E400) zusammen mit 0,3 g Cu(OAc)₂ vorgelegt. Das Gemisch wird bei einem Druck von 20 mbar auf 100-110 °C erwärmt. Mittels einer Taumelkopfpumpe wird die gekühlte Hydroperoxid-Lösung aus a) innerhalb von 2-3 Stunden in die PEG-Lösung dosiert. Parallel hierzu wird ein Azeotrop aus Propionsäure und Wasser kontinuierlich aus der Reaktionslösung abdestilliert. Der auf diese Weise erhaltene Reaktionsaustrag wird zunächst bei 5 mbar und einer Sumpftemperatur von 150 °C über eine einfache Brücke destilliert. Anschließend wird das Destillat aus der Grobdestillation über eine 20 cm Füllkörperkolonne bei einem Druck von 0,5-1 mbar und einer Übergangstemperatur von 90-105 °C feindestilliert. Auf diese Weise können 25 g eines 15-Dimethylpentadecenolid-Gemisches mit einer Reinheit von >95 % erhalten werden. Das 15-Dimethylpentadecenolid-Gemisch enthält die Doppelbindungsisomere: trans-15-Dimethylpentadec-11-en-15-olid
cis-15-Dimethylpentadec-11-en-15-olid
trans-15-Dimethypentadec-12-en-15-olid
cis-15-Dimethylpentadec-12-en-15-olid

### Beispiel 5: Herstellung von 14-Dimethyltetradecanolid - Hydrierung der Doppelbindung

20,6 g 14-Dimethyltetradecenolid aus Beispiel 3 wurden zusammen mit Toluol in einem 250 ml-Dreihalskolben mit einer Gasbürette (1 bar H₂) vorgelegt und auf 95 °C erwärmt. Nach der Zugabe von 0,2 g eines Pd/C-Katalysators wurde die Apparatur durch die Zuschaltung der Gasbürette mit Wasserstoff überlagert. Die Reaktionslösung wurde für ca. 11 h bei 95 °C gerührt, die Wasserstoffaufnahme während dieser Zeit betrug 1085 ml. Anschließend wurde die Reaktionslösung auf Raumtemperatur abgekühlt, der Katalysator abfiltriert und Toluol am Rotationsverdampfer abgezogen. Der Rückstand wurde über eine Füllkörperkolonne bei einem Vakuum von 0,4-0,5 mbar destilliert. Bei einer Übergangstemperatur von 98-100 °C konnte das Produkt 14-Dimethyltetradecanolid mit einer Reinheit von >90 % isoliert werden.

### Beispiel 6: Vorschrift zur Herstellung von 15-Dimethylpentadecanolid - Hydrierung der Doppelbindung

20 g 15-Dimethylpentadecenolid werden zusammen mit Toluol in einem 250 ml-Dreihalskolben mit einer Gasbürette (1 bar H₂) vorgelegt und auf 95 °C erwärmt. Nach der Zugabe von 0,2 g eines Pd/C-Katalysators wird die Apparatur durch die Zuschaltung der Gasbürette mit Wasserstoff überlagert. Die Reaktionslösung wird so lange bei 95 °C gerührt, bis keine Wasserstoffaufnahme mehr zu beobachten ist. Anschließend wird die Reaktionslösung auf Raumtemperatur abgekühlt, der Katalysator abfiltriert und Toluol am Rotationsverdampfer abgezogen. Der Rückstand wird über eine Füllkörperkolonne bei einem Vakuum von 0,4-0,5 mbar destilliert, um das Produkt 15-Dimethylpentadecanolid zu erhalten.

## Patentansprüche

1. Verbindungen der Formeln (I) oder (II) oder Gemische derselben 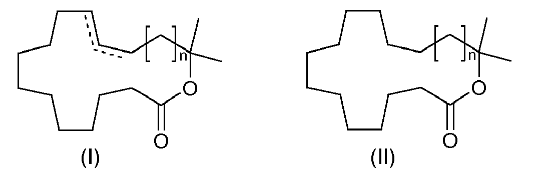 worin
n gleich null (0) oder eins (1) ist und
die gestrichelte Linie eine zusätzliche Doppelbindung in 11- oder 12-Position in cis- oder trans-Konfiguration bedeutet.

2. Verbindungen der Formeln (I) gemäß Anspruch 1, wobei es sich um mindestens eines der Doppelbindungsisomere der Formeln (Ia), (Ib), (Ic) oder (Id) oder um Gemische derselben 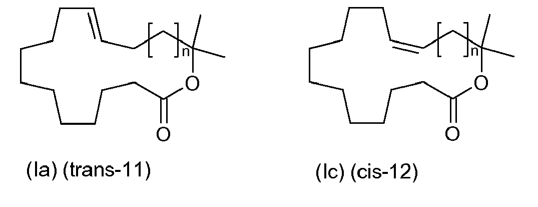 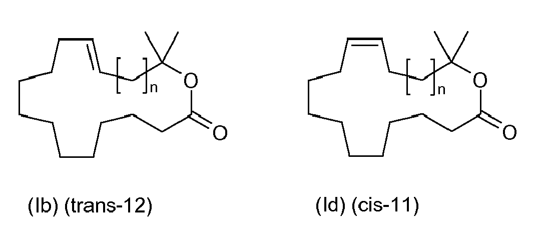 handelt und n wie in Formel (I) definiert ist.

3. Verfahren zur Herstellung der Verbindungen der Formeln (I) oder (II) gemäß Anspruch 1 oder der Verbindungen der Formeln (Ia), (Ib), (Ic) oder (Id) gemäß Anspruch 2 umfassend die Reaktionsschritte:
a) Alkylierung des Cyclododecanons der Formel (III) 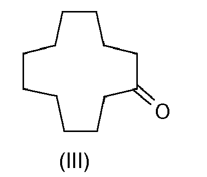 mit einer Verbindung der Formel (IVa) oder einer Verbindung der Formel (IVb) 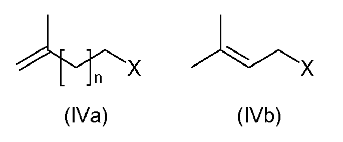 worin in Formel (IVa) und (IVb)
X eine Abgangsgruppe ist und
n gleich null (0) oder eins (1) ist,
zu einer entsprechenden Verbindung der Formel (Va) oder der Formel (Vb), 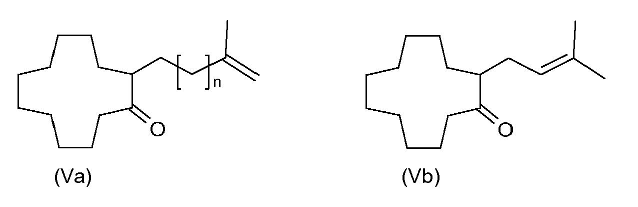 worin in Formel (Va) n gleich null (0) oder eins (1) ist;
b) Cyclisierung einer der Verbindungen der Formeln (Va) oder (Vb) zu einer entsprechenden Verbindung der Formel (VI); 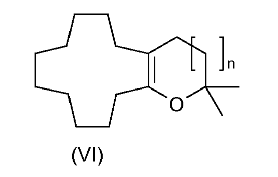 worin in Formel (Vl) n gleich null (0) oder eins (1) ist;
c) Addition von H₂O₂ an die Doppelbindung der Verbindung der Formel (VI) unter Erhalt einer Verbindung der Formel (VII) und anschließende Übergangsmetall [ÜM] - katalysierte Fragmentierung der Verbindung der Formel (VII) zu Verbindungen der Formel (I) 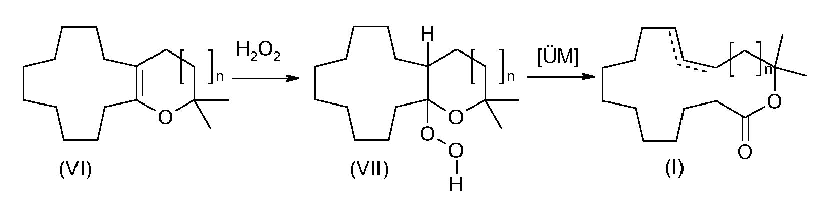 und gegebenenfalls
d) Hydrierung der Verbindungen der Formel (I) zu einer Verbindung der Formel (II) 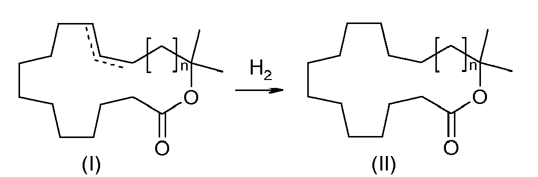 worin in den Formeln (I), (II) und (VII)
n gleich null (0) oder eins (1) ist und
die gestrichelte Linie in Formel (I) eine zusätzliche Doppelbindung in 11- oder 12-Position in cis- oder trans-Konfiguration bedeutet.

4. Verfahren zur Herstellung der Verbindungen der Formeln (I) oder (II) gemäß Anspruch 1 oder der Verbindungen der Formeln (Ia), (Ib), (Ic) oder (Id) gemäß Anspruch 2 umfassend als einen der Reaktionsschritte eine Cyclisierung einer Verbindung der Formel (Va) oder der Formel (Vb), 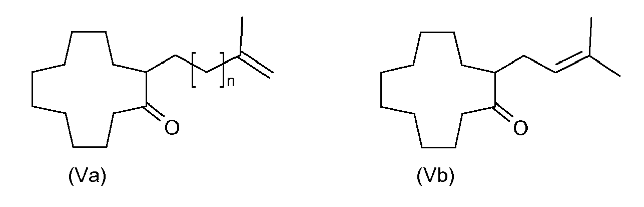 zu einer entsprechenden Verbindung der Formel (VI); 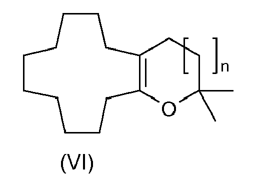 worin in Formeln (Va) und (VI) n gleich null (0) oder eins (1) ist, **dadurch gekennzeichnet, dass** die Cyclisierung in Gegenwart einer Brönstedt- oder Lewis-Säure als Reaktivdestillation durchgeführt wird, wobei die gebildete Verbindung der Formel (Vl) destillativ aus dem Reaktionsgemisch von der Verbindung der Formel (IIIa) oder der Formel (IIIb) abgetrennt wird.

5. Verwendung der Verbindungen der Formeln (I) und/oder (II) gemäß Anspruch 1 oder der Verbindungen der Formeln (Ia), (Ib), (Ic) und/oder (Id) gemäß Anspruch 2 als Riechstoffe.

6. Verwendung der Verbindungen der Formeln (I) und/oder (II) gemäß Anspruch 1 oder der Verbindungen der Formeln (Ia), (Ib), (Ic) und/oder (Id) gemäß Anspruch 2 als Riechstoff zusammen mit mindestens einem weiteren Riechstoff in einer Riechstoffkomposition.

7. Riechstoffkomposition enthaltend mindestens eine der Verbindungen der Formeln (I) und/oder (II) gemäß Anspruch 1 oder der Verbindungen der Formeln (Ia), (Ib), (Ic) und/oder (Id) gemäß Anspruch 2 zusammen mit mindestens einem weiteren Riechstoff.

8. Parfümiertes oder aromatisiertes Produkt oder Artikel enthaltend organoleptisch aktive Mengen mindestens einer der Verbindungen der Formeln (I) und/oder (II) gemäß Anspruch 1 oder der Verbindungen der Formeln (Ia), (Ib), (Ic) und/oder (Id) gemäß Anspruch 2.
